# EUROPEAN PATENT SPECIFICATION

(11) **EP 3 471 651 B1**
(45) Date of publication and mention of the grant of the patent: **11.03.2020**
(21) Application number: 16751354.8
(22) Date of filing: 21.06.2016
(51) Int. Cl.: A61B 90/90, A61B 90/98, A61B 90/00

(54) **IDENTIFICATION TAG ATTACHMENT**
BEFESTIGUNG EINES IDENTIFIKATIONSETIKETTS
ATTACHE D'ÉTIQUETTE D'IDENTIFICATION

(43) Date of publication of application: 24.04.2019
(73) Proprietor: Haldor Advanced Technologies Ltd, 45319 Hod HaSharon (IL)
(72) Inventor: DOR, Guy, 45871 Rosh Haayn (IL); ZEELI, Dan, North York Ontario M3H 4L5 (CA); KADOSH-TAMARI, Ilan, 47204 Ramat Hasharon (IL)
(74) Representative: Marchitelli, Mauro
(86) International application number: PCT/IL2016/050661
(87) International publication number: WO 2017/221227

(56) References cited:
- WO-A1-96/08596
- WO-A1-2012/146867
- WO-A1-2015/086775
- WO-A2-2011/054355
- FR-A1- 2 918 769

## Description

### TECHNICAL FIELD

The present invention relates to the attachment of an identification tag to a tool.

### BACKGROUND

There are many environments in which multiple tools are used, including for example operation rooms, aircraft hangars, garages, or the like.

An operation room is a facility in which intrusive operations are performed on patients. Typically, multiple people participate in an operation, including a chief surgeon, an assistant surgeon, an anesthesiologist, a scrub nurse, and a circulating nurse. The participating personnel use multiple surgical items, such as scalpels, forceps, and other tools, varying according to the surgery being performed.

Intensive efforts are invested in keeping track of all surgical items and disposables, in order to make sure no item unintentionally remains inside the patient's body. Therefore careful counting is performed before, during and after the operation.

Counting the surgical items is a tedious job and requires intensive resources, including mental resources, personnel time and down-time of the operating room. Counting the surgical items towards the end of an operation also increases the time the patient's body is open with the associated risks.

In addition, counting is not always error-free, and in many cases surgical items end up being left within the patient's body, causing severe damage and even death.

Another problem relates to the life cycle of surgical items. For example, reusable surgical items used in an operation have to be cleaned and sterilized or cleaned and disinfected prior to further usage. Other constraints may relate to maintenance operations required for the surgical items, for example, a blade may have to be sharpened after a specific number of operations in which it is used. In another example, surgical items that have been used in an operation performed on a patient with a contagious disease may require extra sterilization and or disinfection before further usage. Making sure that each surgical item is used and maintained properly also imposes expenses and requires resources, including record keeping and tracking, manual labor and the like.

In US patent no. 8,193,938 to Halberthal et al dated June 5, 2012 there is disclosed a computerized system and method for keeping track of tools, wherein each tool is uniquely identified. Identifying the tools is performed using a Radio Frequency (RF) identification transducer tag that is attached to the tools. The use of a computerized system improves the ability to track the tools and reduce system overhead.

Attaching identifying tags to surgical items imposes a number of challenges. The tag needs to adhere to the surgical item for the entire lifetime of the surgical item, the tag should not interfere with the use of the surgical item, the tag should be identifiable regardless of the orientation of the surgical item and even when surrounded by other surgical items.

US 2015/0317555 A1 ( Application no. 14/269,155 to Guy Dor et al dated May 4, 2014) discloses construction of an identification tag and attaching it to a tool. Generally the attachment process is performed by laser welding to prevent damage to the tag from excess heating. The welding process generally requires that a skilled person perform the attachment process to prevent damage to the tag while being attached.

The current attaching process is time consuming and expensive. In an environment with many tools/surgical items a lot of skilled technicians may be required for attaching identification tags to the tools. Thus it is of interest to reduce the attaching time and simplify it so that even a non-skilled worker may quickly attach identification tags.

It should be noted that government regulatory authorities (e.g. the FDA) are in the process of introducing regulatory requirement requiring medical facilities to permanently mark tools with unique device identifiers (UDI). Thus it is even further of interest to simplify the attachment process to reduce the overhead and risk of not being in compliance with the regulations.

FR 2 918 769 A1 discloses a radio frequency identification marker with a radio-opaque substance.

WO 2011/054355 A2 discloses a method for applying marking elements on an object; an object comprising a marking element and use of a special material for applying a marking element on an object.

WO 2012/146867 A1 discloses a medical device provided with an innovative marking system enabling rapid, repeated and remote identification.

WO 2015/086775 A1 discloses an attachment and cover for an electronic identification tag.

WO 96/08596 A1 discloses a labelling device for laundry items.

### SUMMARY

The invention is disclosed by independent claims 1 and 7, with preferred embodiments disclosed in the dependent claims. An aspect of an embodiment of the disclosure relates to an identification tag for attaching to an item (e.g. a surgical tool). The identification tag is formed as an enclosure with an electronic circuit embedded therein and designed for being adhesively attached to the item. The enclosure is formed from a curable material and the electronic circuit is designed to provide a unique ID in response to a wireless query in the form of an electromagnetic signal. The enclosure is created and attached according to the following:
1) A base that serves as a container is cast from the curable material (e.g. using a mold);
2) The electronic circuit is placed in the base;
3) A second layer of curable material is used to fill around the electronic circuit to make a robust enclosure and to form a protective layer over the electronic circuit; wherein a void is left in the base above the protective layer over the electronic circuit;
4) The robust enclosure is attached to the item by injecting a third layer of curable material in the void and curing it while in contact with the item. The void has a height of at least 0.5 mm to assure that enough curing material is used to connect between the enclosure and the item and that a distance of more than 0.5mm remains between the item and the electronic circuit to prevent adverse effects on the quality of the transmissions to and from the electronic circuit.

There is thus provided according to an exemplary embodiment of the disclosure, an identification tag that is attachable to an item and serves to identify the item with a computerized system, comprising:
a base in the form of a container that is cast from a curable material;
an electronic circuit that is placed in the base and wirelessly provides a unique ID in response to an electromagnetic signal;
a second layer of the curable material filling the base and forming a protective cover over the electronic circuit; and
wherein a void of at least 0.5mm is formed in the base above the second layer; wherein the void is designed to receive a third layer of the curable material for adhesively attaching the identification tag to the item leaving a distance of more than 0.5 mm between the electronic circuit and the item.

In an exemplary embodiment of the disclosure, the height of the void is selected according to the material of the item to which the identification tag is to be attached. Optionally, the base is cured before providing the second layer. In an exemplary embodiment of the disclosure, the second layer is cured before attaching the identification tag to the item. Optionally, the protective cover is thinner than 1 mm. In an exemplary embodiment of the disclosure, the electronic circuit is an RFID circuit.

There is further provided according to an exemplary embodiment of the disclosure, a method of forming an identification tag that is attachable to an item and serves to identify the item with a computerized system, comprising:
casting a base in the form of a container using a curable material;
placing an electronic circuit in the base, wherein the electronic circuit wirelessly provides a unique ID in response to an electromagnetic signal;
filling the base with the curable material to form a second layer; wherein the second layer forms a protective cover over the electronic circuit; and
wherein a void of at least 0.5 mm is formed in the base above the second layer; wherein the void is designed to receive a third layer of the curable material for adhesively attaching the identification tag to the item leaving a distance of more than 0.5 mm between the electronic circuit and the item.

In an exemplary embodiment of the disclosure, the height of the void is selected according to the material of the item to which the identification tag is to be attached. Optionally, the base is cured before providing the second layer. In an exemplary embodiment of the disclosure, the second layer is cured before attaching the identification tag to the item. Optionally, the protective cover is thinner than 1 mm. In an exemplary embodiment of the disclosure, the electronic circuit is an RFID circuit.

### BRIEF DESCRIPTION OF THE DRAWINGS

The present disclosure will be understood and better appreciated from the following detailed description taken in conjunction with the drawings. Identical structures, elements or parts, which appear in more than one figure, are generally labeled with the same or similar number in all the figures in which they appear. It should be noted that the elements or parts in the figures are not necessarily shown to scale and each elements or part may be larger or smaller than actually shown.
Fig. 1 is a schematic illustration of a surgical item with an identification tag and an environment for using the surgical item, according to an exemplary embodiment of the disclosure;
Fig. 2 is a schematic illustration of an exploded view of a surgical item with an identification tag, according to an exemplary embodiment of the disclosure;
   and
Fig. 3 is a flow diagram of a method of attaching an identification tag according to an exemplary embodiment of the disclosure.

### DETAILED DESCRIPTION

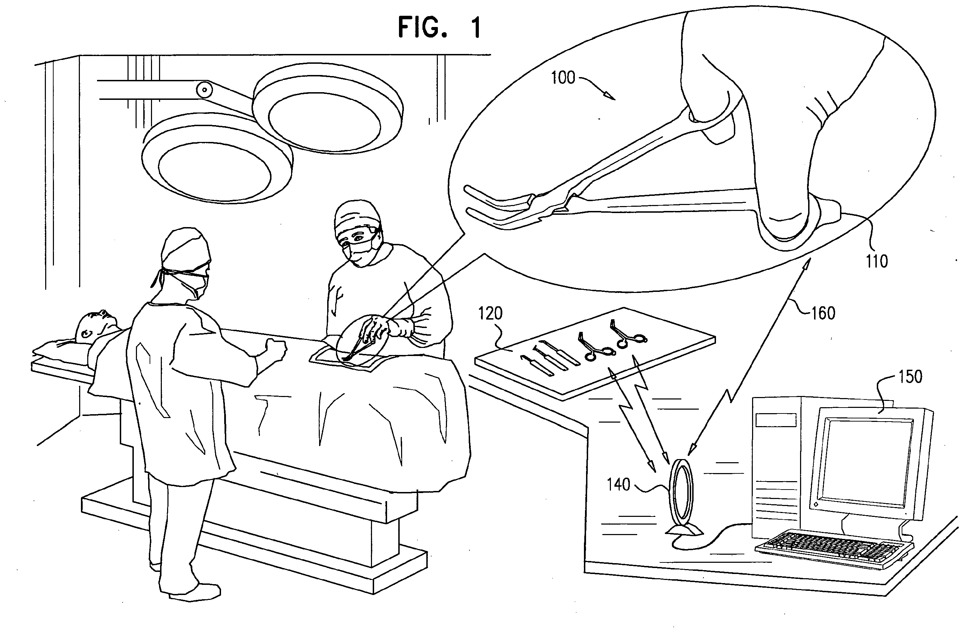

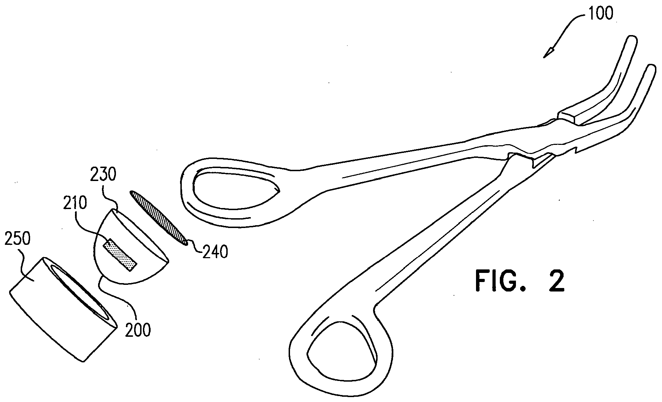

Fig. 1 is a schematic illustration of a surgical item 100 with an identification tag 110 and an environment for using the surgical item 100, according to an exemplary embodiment of the disclosure. In an exemplary embodiment of the disclosure, surgical item 100 is part of a set of items 120 used to perform an operation. Optionally, each item of the set 120 is marked by attaching an identification tag 110. In an exemplary embodiment of the disclosure, the identification tag 110 is made from a curable adhesive material (e.g. epoxy) surrounding an electronic circuit 210 (e.g. an RFID circuit) as shown in Fig. 2. Optionally, the electronic circuit 210 provides a unique ID for identifying each of the surgical items 110. In an exemplary embodiment of the disclosure, the identification tags 110 are provided to a user with the electronic circuit 210 already enclosed within the identification tag 110 for attaching to the surgical items 100 with the same curable adhesive material used to enclose the electronic circuit 210.

In an exemplary embodiment of the disclosure, an antenna 140 is used to communicate wirelessly 160 with identification tags 110 in the vicinity of the antenna 140. Optionally, antenna 140 is attached to a computer 150 that is programed to keep track of the location of the surgical items 100 of set 120 and/or the maintenance processes applied to a specific surgical item 100. Optionally, computer 150 may provide an indication (e.g. audio or visual) when all the identification tags 110 of a specific set 120 have been located, or a notification if a specific surgical item 100 from set 120 has not been located.

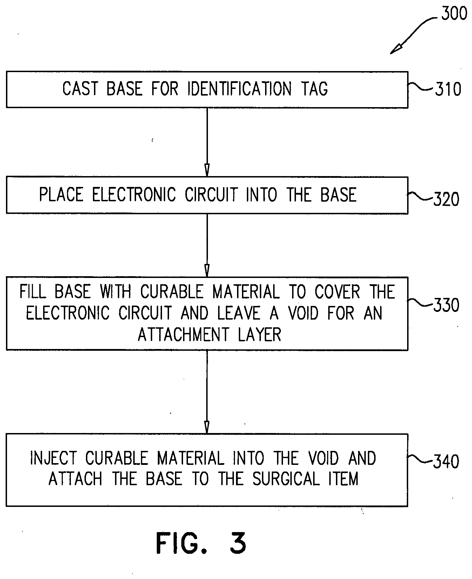

Fig. 2 is a schematic illustration of an exploded view of surgical item 100 with identification tag 110, and Fig. 3 is a flow diagram of a method 300 of attaching identification tag 110 to the surgical item 100, according to an exemplary embodiment of the disclosure. In an exemplary embodiment of the disclosure, identification tag 110 is formed and attached to the surgical item 100 using electronic circuit 210 and three layers of the same curable material. Optionally, the first layer is formed by casting (310) a base 200 with a mold 250, for example by pouring the curable material into the mold and waiting for it to harden. Optionally, the base is formed as a container for example shaped as a semi-sphere or other shape having a hollow inside for placing the electronic circuit 210. Once the base 200 is formed electronic circuit 210 is placed (320) inside the base 200. Then the base 200 is filled (330) with the second layer of curable material to fill the base and cover the electronic circuit 210 forming a robust identification tag 110 for attaching to the surgical item 100.

In an exemplary embodiment of the disclosure, the second layer is designed to cover the electronic circuit 210 with a thin protective layer, for example with a thickness of less than 1mm or less than 2-3 mm. In an embodiment of the disclosure, the second layer is applied such that it leaves a void 230 in the base 200 of the identification tag 110 with a height of at least 0.5 mm or more above the second layer for injecting (340) an attachment layer 240 to adhesively attach the base 200 with the electronic circuit 210. Optionally, after curing the second layer of the identification tag 110, it is provided in this form to the user for attaching to surgical items 100, for example at a health facility (e.g. hospital). In an exemplary embodiment of the disclosure, the height of the void is selected to ensure that the electronic circuit 210 is distanced by at least 0.5 mm away from the surgical item to minimize influence of the surgical item 100 on the reception of the electronic circuit 210. In some embodiments of the disclosure, the size of the void is selected based on the material of the surgical item 100, for example a deeper void may be provided for attaching to metallic surgical items that have a stronger influence on the impedance of the antenna in contrast to non-metallic surgical items.

In an embodiment of the disclosure, the user injects (340) a third layer of curable material into the void 230 and presses the identification tag 110 against the surgical item 100 to attach them together. Optionally, the attachment point on the surgical item 100 is cleaned with alcohol to prepare it for the attachment process.

In an embodiment of the disclosure, the same curable material is used for all the layers to enhance durability of the identification tag, since different materials tend to form weaker connection points.

In some embodiments of the disclosure, heat is applied to enhance the curing speed of the curable material. Alternatively or additionally, UV radiation is used to cure the curing material.

It should be noted that the above description describes mainly attaching tags to surgical items in a medical environment, however it should be noted that the disclosed apparatus and method are applicable also for other types of tools and items in other environments such as factories, garages, and maintenance facilities in general.

It should be appreciated that the above described methods and apparatus may be varied in many ways, including omitting or adding steps, changing the order of steps and the type of devices used. It should be appreciated that different features may be combined in different ways. In particular, not all the features shown above in a particular embodiment are necessary in every embodiment of the disclosure. Further combinations of the above features are also considered to be within the scope of some embodiments of the disclosure. It will also be appreciated by persons skilled in the art that the present disclosure is not limited to what has been particularly shown and described hereinabove.

The scope of the present invention is defined by the appended claims.

## Claims

1. An identification tag (110) that is attachable to an item (100) and serves to identify the item (100) with a computerized system (150), comprising:
a base (200) in the form of a container that is cast from a curable material;
an electronic circuit (210) that is placed in the base (200) and wirelessly provides a unique ID in response to an electromagnetic signal;
**characterized by**:
a second layer of said curable material filling the base (200) and forming a protective cover over the electronic circuit (210); and
wherein a void (230) of at least 0.5mm is formed in the base (200) above the second layer; wherein the void (230) is designed to receive a third layer (240) of said curable material for adhesively attaching the identification tag (110) to the item (100) leaving a distance of more than 0.5mm between the electronic circuit (210) and the item (100).

2. An identification tag (110) according to claim 1, wherein the height of the void (230) is selected according to the material of the item to which the identification tag (110) is to be attached.

3. An identification tag (110) according to claim 1, wherein the base (200) is cured before providing the second layer.

4. An identification tag (110) according to claim 1, wherein the second layer is cured before attaching the identification tag (110) to the item (100).

5. An identification tag (110) according to claim 1, wherein the protective cover is thinner than 1mm.

6. An identification tag (110) according to claim 1, wherein the electronic circuit (210) is an RFID circuit.

7. A method of forming an identification tag (110) that is attachable to an item (100) and serves to identify the item with a computerized system (150), comprising:
casting a base (200) in the form of a container using a curable material;
placing an electronic circuit (210) in the base (200), wherein the electronic circuit (210) wirelessly provides a unique ID in response to an electromagnetic signal;
**characterized by**:
filling the base (200) with said curable material to form a second layer ;
wherein the second layer forms a protective cover over the electronic circuit (210); and
wherein a void (230) of at least 0.5mm is formed in the base (200) above the second layer; wherein the void (230) is designed to receive a third layer (240) of said curable material for adhesively attaching the identification tag (110) to the item (100) leaving a distance of more than 0.5mm between the electronic circuit (210) and the item (100).

8. A method according to claim 7, wherein the height of the void (230) is selected according to the material of the item (100) to which the identification tag (110) is to be attached.

9. A method according to claim 7, wherein the base (200) is cured before providing the second layer.

10. A method according to claim 7, wherein the second layer is cured before attaching the identification tag (110) to the item (100).

11. A method according to claim 7, wherein the protective cover is thinner than 1mm.

12. A method according to claim 7, wherein the electronic circuit (210) is an RFID circuit.

## Patentansprüche

1. Identifikations-Tag (110), das an einem Objekt (100) angebracht werden kann und dazu dient, das Objekt (100) mit einem computergestützen System (150) zu identifizieren, umfassend:
eine Basis (200) in der Form eines Behälters, der aus einem härtbaren Material gegossen ist;
eine elektronische Schaltung (210), die in der Basis (200) angeordnet ist und als Reaktion auf ein elektromagnetisches Signal drahtlos eine eindeutige ID bereitstellt;
**gekennzeichnet durch**:
eine zweite Schicht des härtbaren Materials, die die Basis (200) füllt und eine Schutzabdeckung über der elektronischen Schaltung (210) bildet; und
wobei ein Hohlraum (230) von mindestens 0,5 mm in der Basis (200) über der zweiten Schicht ausgebildet ist; wobei der Hohlraum (230) dahingehend ausgestaltet ist, eine dritte Schicht (240) des härtbaren Materials zum Ankleben des Identifikations-Tags (110) an dem Objekt (100) aufzunehmen, wobei ein Abstand von mehr als 0,5 mm zwischen der elektronischen Schaltung (210) und dem Objekt (100) belassen wird.

2. Identifikations-Tag (110) nach Anspruch 1, wobei die Höhe des Hohlraums (230) gemäß dem Material des Objekts, an dem das Identifikations-Tag (110) anzubringen ist, gewählt ist.

3. Identifikations-Tag (110) nach Anspruch 1, wobei die Basis (200) ausgehärtet wird, bevor die zweite Schicht bereitgestellt wird.

4. Identifikations-Tag (110) nach Anspruch 1, wobei die zweite Schicht ausgehärtet wird, bevor das Identifikations-Tag (110) an dem Objekt (100) angebracht wird.

5. Identifikations-Tag (110) nach Anspruch 1, wobei die Schutzabdeckung dünner als 1 mm ist.

6. Identifikations-Tag (110) nach Anspruch 1, wobei es sich bei der elektronischen Schaltung (210) um eine RFID-Schaltung handelt.

7. Verfahren zum Ausbilden eines Identifikations-Tags (110), das an einem Objekt (100) angebracht werden kann und dazu dient, das Objekt mit einem computergestützen System (150) zu identifizieren, umfassend:
Gießen einer Basis (200) in der Form eines Behälters unter Verwendung eines härtbaren Materials;
Anordnen einer elektronischen Schaltung (210) in der Basis (200), wobei die elektronische Schaltung (210) als Reaktion auf ein elektromagnetisches Signal drahtlos eine eindeutige ID bereitstellt;
**gekennzeichnet durch**:
Füllen der Basis (200) mit dem härtbaren Material, um eine zweite Schicht zu bilden; wobei die zweite Schicht eine Schutzabdeckung über der elektronischen Schaltung (210) bildet; und
wobei ein Hohlraum (230) von mindestens 0,5 mm in der Basis (200) über der zweiten Schicht ausgebildet wird; wobei der Hohlraum (230) dahingehend ausgestaltet ist, eine dritte Schicht (240) des härtbaren Materials zum Ankleben des Identifikations-Tags (110) an dem Objekt (100) aufzunehmen, wobei ein Abstand von mehr als 0,5 mm zwischen der elektronischen Schaltung (210) und dem Objekt (100) belassen wird.

8. Verfahren nach Anspruch 7, wobei die Höhe des Hohlraums (230) gemäß dem Material des Objekts (100), an dem das Identifikations-Tag (110) anzubringen ist, gewählt wird.

9. Verfahren nach Anspruch 7, wobei die Basis (200) ausgehärtet wird, bevor die zweite Schicht bereitgestellt wird.

10. Verfahren nach Anspruch 7, wobei die zweite Schicht ausgehärtet wird, bevor das Identifikations-Tag (110) an dem Objekt (100) angebracht wird.

11. Verfahren nach Anspruch 7, wobei die Schutzabdeckung dünner als 1 mm ist.

12. Verfahren nach Anspruch 7, wobei es sich bei der elektronischen Schaltung (210) um eine RFID-Schaltung handelt.

## Revendications

1. Étiquette d'identification (110) qui peut être fixée à un article (100) et qui sert à identifier l'article (100) avec un système informatisé (150), l'étiquette d'identification comprenant :
une base (200) sous la forme d'un conteneur qui est coulé à partir d'un matériau durcissable ;
un circuit électronique (210) qui est placé dans la base (200) et qui fournit sans fil un ID unique en réponse à un signal électromagnétique ;
l'étiquette d'identification étant **caractérisée par** :
une deuxième couche dudit matériau durcissable remplissant la base (200) et formant un revêtement protecteur sur le circuit électronique (210) ; et
un vide (230) d'au moins 0,5 mm étant formé dans la base (200) au-dessus de la deuxième couche ;
le vide (230) étant conçu pour recevoir une troisième couche (240) dudit matériau durcissable afin de fixer de manière adhésive l'étiquette d'identification (110) à l'article (100), en laissant une distance de plus de 0,5 mm entre le circuit électronique (210) et l'article (100).

2. Étiquette d'identification (110) selon la revendication 1, dans laquelle la hauteur du vide (230) est sélectionnée selon le matériau de l'article auquel l'étiquette d'identification (110) doit être fixée.

3. Étiquette d'identification (110) selon la revendication 1, dans laquelle la base (200) est durcie avant d'appliquer la deuxième couche.

4. Étiquette d'identification (110) selon la revendication 1, dans laquelle la deuxième couche est durcie avant de fixer l'étiquette d'identification (110) à l'article (100).

5. Étiquette d'identification (110) selon la revendication 1, dans laquelle le revêtement protecteur est plus fin que 1 mm.

6. Étiquette d'identification (110) selon la revendication 1, dans laquelle le circuit électronique (210) est un circuit RFID.

7. Procédé de formation d'une étiquette d'identification (110) qui peut être fixée à un article (100) et qui sert à identifier l'article avec un système informatisé (150), le procédé consistant à :
couler une base (200) sous la forme d'un conteneur au moyen d'un matériau durcissable ;
placer un circuit électronique (210) dans la base (200), le circuit électronique (210) fournissant sans fil un ID unique en réponse à un signal électromagnétique ;
le procédé étant **caractérisé par** l'étape consistant à :
remplir la base (200) avec ledit matériau durcissable pour former une deuxième couche ;
la deuxième couche formant un revêtement protecteur sur le circuit électronique (210) ; et
un vide (230) d'au moins 0,5 mm étant formé dans la base (200) au-dessus de la deuxième couche ;
le vide (230) étant conçu pour recevoir une troisième couche (240) dudit matériau durcissable afin de fixer de manière adhésive l'étiquette d'identification (110) à l'article (100), en laissant une distance de plus de 0,5 mm entre le circuit électronique (210) et l'article (100).

8. Procédé selon la revendication 7, dans lequel la hauteur du vide (230) est sélectionnée selon le matériau de l'article (100) auquel l'étiquette d'identification (110) doit être fixée.

9. Procédé selon la revendication 7, dans lequel la base (200) est durcie avant d'appliquer la deuxième couche.

10. Procédé selon la revendication 7, dans lequel la deuxième couche est durcie avant de fixer l'étiquette d'identification (110) à l'article (100).

11. Procédé selon la revendication 7, dans lequel le revêtement protecteur est plus fin que 1 mm.

12. Procédé selon la revendication 7, dans lequel le circuit électronique (210) est un circuit RFID.
